# EUROPEAN PATENT SPECIFICATION

(11) **EP 2 168 584 B1**
(45) Date of publication and mention of the grant of the patent: **20.08.2014**
(21) Application number: 08778975.6
(22) Date of filing: 04.07.2008
(51) Int. Cl.: A61K 31/5517, A61K 31/4525, A61P 25/24

(54) **PHARMACEUTICAL COMPOSITION COMPRISING THE COMBINATION OF A TRIAZOLOBENZODIAZEPINE AND A SELECTIVE SEROTONIN REUPTAKE INHIBITOR**
PHARMAZEUTISCHE ZUSAMMENSETZUNG MIT EINER KOMBINATION AUS EINEM TRIAZOLOBENZODIAZEPIN UND EINEM SELEKTIVEN SEROTONIN-WIEDERAUFNAHMEHEMMER
COMPOSITION PHARMACEUTIQUE COMBINANT UNE TRIAZOLOBENZODIAZÉPINE ET UN AGENT INHIBITEUR SÉLECTIF DE LA RECAPTURE DE LA SÉROTONINE

(30) Priority: 06.07.2007 MX MX07008321
(43) Date of publication of application: 31.03.2010
(73) Proprietor: PPTM INTERNATIONAL S.à r.l., 1253 Luxembourg (LU)
(72) Inventor: GARCÍA ARMENTA, María Elena, Jalisco (MX); SANTOS MURILLO, Josefina, Jalisco (MX); ÁLVAREZ OCHOA, Víctor Guillermo, C.P. 45222 Zapopan, Jalisco (MX); ESPINOZA ABDALA, Leopoldo de Jesus, C.P. 45110 Zapopan, Jalisco (MX)
(74) Representative: Carvajal y Urquijo, Isabel
(86) International application number: PCT/MX2008/000085
(87) International publication number: WO 2009/008696

(56) References cited:
- CALVO G. ET AL.: 'Lack of pharmacologic interaction between paroxetine and alprazolam at steady state in healthy volunteers' JOURNAL OF CLINICAL PHARMACOLOGY vol. 24, no. 3, 2004, pages 268 - 276, XP009133923
- TANII H. ET AL.: 'Possible neuroleptic malignant syndrome related to concomitant treatment with paroxetine and alprazolam' PROGRESS IN NEURO-PSYCHOPHARMACOLOGY & BIOLOGICAL PSYCHIATRY vol. 30, 2006, pages 1176 - 1178, XP008126537
- GALLELLI L. ET AL.: 'Generalised urticaria in a young women treated with clomipramine and after ingestion of codfish' PHARMACOPSYCHIATRY vol. 39, no. 4, 2006, pages 154 - 146, XP008126549
- LORENZO J.L.: 'Changes in dream recall and dream content under drug treatment in healthy subjects: effects of alprazolam, paroxetine, and its combination (0-1)' JOURNAL OF SLEEP RESEARCH vol. 11, no. SUPPL. 1, 2002, page 139, XP008126550

## Description

### FIELD OF THE INVENTION

The present invention is to be applied in the pharmaceutical industry and describes a pharmaceutical composition formed by the synergistic combination of an agent belonging to the triazolobenzodiazepine group, such as the active principle: Alprazolam and a selective serotonin reuptake inhibitor agent, such as the active principle: Paroxetine hemihydrate; which are formulated in a single dosing unit, said unit being prescribed for the treatment and control of major depressive episodes, Obsessive-Compulsive disorder (OCD), panic attacks with or without agoraphobia, generalized anxiety disorders and social phobia and post-traumatic stress disorder.

The combination of the active principles above produces a major synergistic effect when administered simultaneously in a single dosing unit, as opposed to when these are administrated alone, providing benefits such as: reduced concentrations of at least one of the active principles formulated, reduced administered doses, quicker action, increased therapeutic effect efficacy, reduced treatment administration periods and reduced risk of side effects, including: Dependence on the drugs comprised in the formulation and interaction between the active principles, which could cause hepatic damage.

### BACKGROUND OF THE INVENTION

Nowadays, the World Health Organization (WHO) defines the Major Depressive Disorder as the fourth disease on the list of the most important public health problems all over the world. The consequences of depression are comparable to or exceed those produced by: Arterial hypertension, Pain, Diabetes mellitus and Coronary Disease. Mortality and morbidity of these pathologies increase if there is co-morbidity with depression.

Major depression constitutes a public health problem, due to its high morbidity: 5% of the world population suffers from some depressive disorder. This pathology can evolve in an undesirable way, causing severe consequences such as suicide.

*Major Depressive Disorder* occurs mainly in individuals older than 40 years, starting approximately from 25 (50%) and 40 years of age (earlier in female subjects). In individuals older than 60 years, there is a smaller proportion of first depressive episodes (10%).

Even though major depression is he most prevalent medical disorder all over the world and it can be efficiently treated through different interventions, such as, in most of cases, identifying it (specially by psychiatrists) and despite of the governmental investments intended for its adequate control and prevention, these effort are minimal when compared to other actions accomplished for less frequent or less treatable diseases.

The etiology of depression is complex, as it involves several genetic, biologic and psychosocial factors. Among the biologic factors, there is evidence of alterations in terms of neurotransmitters, cytokines and hormones, besides of modifications in the nervous, immune and endocrine systems.

Psychoneuroimmunology has shown the presence of cytokine-mediated alterations in the hypothalamus-hypophysis-adrenal axis; as well as immunologic alterations associated with the neurotransmitters present in major depression, including a reduction of the number of serotonin transporters (5-TH: 5-Hidroxitriptophan) in peripheral blood cells of depressed patients. This shows a direct relationship between serotonin and the immunologic system in this kind of affective disorders.

The specific cell immune response is affected by the presence of serotonin, as peripheral blood cells have receptors and transporters for this neurotransmitter, which in turn, is related to psychiatric disorders, particularly, to depression.

Regarding serotonin receptors (5-HT), it is necessary to mention that there are 7 subtypes of receptors: 5-HT1, 5-HT2, 5-HT3, 5-HT4, 5-HT5, 5-HT6 and 5-HT7. In particular, the 5HT1A subtype mediates the regulation of brain functions, such as sexual behavior, development of depressive episodes, state of aggression and orthostatic hypertension, among others. Such receptor is characterized by the use of ligands marked with radioactive isotopes, such as [3H]-buspirone and [3H]-espiperone. Serotonin acts on pre- and post-synaptic receptors once it is in the synaptic cleft, being removed from this space through serotonin reuptake, which prevents the progression of neurotransmission.

Serotonin transporters (5-HT) can carry out sodium (Na+)-dependent secondary active transport. These transporters are present in peripheral blood cells, and can be expressed as well in central nervous system neurons, platelets, placenta, pulmonary endothelium and mastocytes. Evidence exist that the properties of the serotonin transporter present in peripheral blood cells are similar to those of the 5HT transporter present in the neuronal tissue. This is an active transport system showing dependence on Sodium (Na+) and temperature. When Na+ is replaced by choline ions, the 5HT-reuptake levels decrease by 90%. According to the sensibility to anti-depressant drugs, two types of transporters have been described: The first one is in the cell plasma membrane and the second one is in the cytoplasmic vesicles of human peripheral blood cells, the latter being the most sensible cytoplasmic membrane transporter. Depression is a syndrome characterized by a state of sadness and unhappiness, which can be transient or permanent. In most cases, individuals describe their state -and it is perceived in this way by others- as low mood, decreased potentiality, and lack of affection, usual axis of action, poor interest, and hypoergy. Furthermore, depression comprises a range of clinical variants according to its evolution, treatment response, and tendency to chronicity, recurrence and severity, which can appear as a mild disorder until these evolve into psychotic symptoms.

Depressive disorders belong to the taxonomical category of affective disorders. In turn, these are subdivided in three groups: major depressive disorder, dysthymic disorder and nonspecific depressive disorder. Among these, major depression (also called unipolar depression) and dysthymia are the most frequent categories, representing one of the main psychiatric and mental health problems nowadays. The DSM (Diagnostic and Statistical Manual of Mental Disorders of the American Psychiatric Association) defines the major depressive disorder as the presence of one or more major depressive episodes without a history of maniac, hypomaniac or mixed episodes. A major depressive episode most last at least two weeks and it is characterized by the appearance of symptoms such as: pathological sadness, anxiety, weakness and anhedonia (low mood most of the day, almost everyday and lack of interest or inability to feel pleasure in almost all activities); appetite alterations and weight alterations (weight loss or increase); sleep alterations (insomnia or hypersomnia); fatigue or energy loss (unjustified tiredness, sometimes with variations during the day, which frequently increase in the morning); thought alterations (subjects think they suffer from all kind of diseases, defeatist ideas, obsessions, monotonous and slow thoughts, weaken memory and frequent distraction); somatic alterations (chronic or erratic pain, as well as constipation and night sweating); behavioral alterations (isolation, experimenting states of tranquility at moments and exploding later in violent angst crisis o crying attacks for insignificant reasons, which makes it difficult to make decisions); restlessness or lessening of psychomotor activity; feelings of extreme disadvantage and/or guilt; decreased capacity to think or concentrate; recurrent death or suicidal thoughts.

The manifestation of such symptoms affects the patient in social and work terms. It is highly important than the specialist physician ensures these symptoms are not caused by the presence of physiological effects, such as the consumption of substances including: drugs, medicines, alcoholic drinks, etc; the presence of mourning or a medical condition.

Usually, major depressive disorder presents a chronic course and subjects tend to have relapses. The chronic forms of the disease develop in 15 to 20% of patients, with women being the most affected population.

From 75% to 80% of patients suffering from major depressive disorder have relapses, and non-recurrent remote depressive episodes are not frequent in prospective and longitudinal studies in patients.

This condition can evolve in an undesirable way, causing severe consequences such as suicide and socio-family dysfunction. Suicide occurs in 10 to 20% of patients suffering from major depression. However, risk index can reach up to 20% if an appropriate diagnostic or treatment of the disease is not provided.

By 2020, major depression will become the second most disabling condition, surpassed only by ischemic heart disease. Depression varies depending on gender, being more frequent in women (25%) than in men (12%). Severe depression can develop at any age, but the average appearance period is about 25 years of age.

In regard to the diagnostic of major depressive disorder, it is known that, in terms of the response and severity of this condition: a) previous depressive symptoms, which even constitute a nosologic entity, are a predisposing factor for presenting major depressive disorder in more than 50% of cases; b) the greater the number of depressive episodes, the greater is the risk and severity of relapses; c) a current severe or prolonged episode (mainly in elderly patients) is frequently accompanied by treatment refractoriness (mortality index caused by major depressive disorder is much greater in individuals older then 55 years); d) the shorter the lapse from the beginning of the episode to the beginning of the treatment, the higher recovery probabilities; similarly, the fewer the changes applied to the initial drug treatment, the higher the probabilities of being free from recurrences; e) co-morbid Obsessive-Convulsive Disorders (OCD) which implies a worse prognostic, but it decreases the risk of suicide; f) the absence maintenance treatment increases the risk of relapse and recurrence; g) personality disorders, double depression and high number of previous hospitalizations contribute to a inappropriate response to conventional anti-depressant treatments.

Because of the above, its integral understanding will allow finding better therapeutic tools to prevent the consequences of major depression, increasing the patient life quality and making its prognostic more favorable.

Usually, *Obsessive-Compulsive Disorder* (OCD) starts during adolescence or early adulthood. OCD is characterized by obsessions and/or recurrent compulsions intense enough for causing severe discomfort.

Obsessions are undesired recurrent and persistent thoughts, impulses or images causing high levels of angst or anxiety. Usually, these are irrational or unreal, not just exaggerated concerns about real life problems. Individuals try to ignore or repress these thoughts, impulses or images, or tries to neutralize he same by other thoughts or activities.

Compulsions are repetitive or ritual behavior (such as washing hands, gathering objects, placing objects in a given order, checking on something) or mental acts (like counting, repeating words in silence, avoiding things or actions), which the individual must carry out in response to an obsession for certain rules that must be followed.

The objective of these behaviors or mental actions is to avoid or reduce discomfort or to avoid some negative event or situation; however, these behaviors or mental actions do not have a real connection with the thing they want to neutralize or avoid, or these are clearly excessive. At some point during the disorder course, the individual recognizes that these obsessions or compulsions are excessive or irrational.

The obsessions or compulsions cause significant anxiety or angst, representing waste of time, besides the fact that these noticeably interfere with the usual routine, work performance, social activities or interpersonal relationships of the individual. Obsessive thoughts vary depending on the age of the individual and these can change throughout the time. Research shows that OCD is a brain disorder, which tends to be repetitive among certain families, but it does not mean the individual must present symptoms.

*Panic Disorder* is a rather frequent and treatable disorder. Individuals suffering from panic disorder present unexpected and repeated periods of intense terror or discomfort, accompanied by other symptoms, such as: fast heartbeats and shortness of breath. Such periods are called "panic attacks" lasting from a few minutes to several hours.

Panic attacks occur without previous notice. The symptoms of panic attacks are as follows: intense terror (the feeling that something terrible will happen); fast heartbeats or tachycardia; dizziness or vertigo; shortness of breath or suffocation; shaking o tremors; sensation of unreality; fear of dying or losing control over certain situation or going crazy.

Usually, panic disorder starts during adolescence, but it can start during childhood. If it is not identified and treated, panic disorder and its complications can be devastating. Panic attacks can interfere with social relationships, work activities and normal development of the individual. Individuals suffering from panic disorder can start to feel anxious most of the time, even when they are not having a panic attack. Some individuals start avoiding situations that could produce a panic attack or situations where there is no help available. This pattern, which consists of avoiding certain places or situations, is called agoraphobia. Some individuals suffering from panic attacks can develop a major depression and can be at risk of presenting suicidal behavior. In an attempt to reduce anxiety, some individuals suffering from panic disorder use alcohol or drugs as an alternative.

The *Anxiety Disorder* is an emotional reaction to the perception of a threat or danger and is purpose is protecting the individual. All mammals have genetically programmed adaptive biological mechanisms, which purpose is to protect their offspring and themselves against potential harm. In human beings, when there is an unexpected or intense such as an explosion, screams, or seeing frightened people escaping, an anxiety response is produced automatically and immediately, which prepares the individual o fight or flee. If the stimulus is rather intense and terrifying, the response will consist of remaining immobile or paralyzed. The terms angst, nervousness, insecurity, restlessness, stress, apprehensiveness or fear are for describing different experiences related to anxiety.

Anxiety is a state of psychophysical discomfort characterized by a sensation of restlessness, intranquility, insecurity or uneasiness in response to a non-specific-cause imminent threat. The main difference between normal anxiety and pathological anxiety is that the later is based on an unreal or distorted perception of such threat. When anxiety is quite severe and acute, it can paralyze he individual, turning into panic.

Anxious responses become evident through a series of manifestations, which can be grouped in tree systems or areas: 1.- Physiological, somatic or body manifestations; 2.-Cognitive, mental o subjective manifestations and 3.-Behavioral manifestations. While these three systems become evident as a whole, they can be activated in a greater or lesser degree in different individuals. This means that each individual have a particular way of reacting, in which any of the above responses can be more evident than others, or a tripartite response can occur in a more or less proportional way.

The physiological, somatic or neurovegetative symptoms of Anxiety are produced by the hyperactivity of the autonomous nervous or neurovegetative system, especially that of the sympathetic nervous system, which generates alarm responses and are manifested in the different systems forming the human body. The cardiovascular symptoms are: tachycardia or accelerated pulse, increased blood pressure, palpitations, chest oppression, paleness or blush; the respiratory symptoms are: suffocation or shortness of breath, accelerated and superficial respiratory rhythm, dyspnoea or labored breathing; gastrointestinal symptoms are: nausea, choking sensation or difficulty to swallow, vomit, diarrhea and intestinal cramps, flatulence or gases, aerophobia or inhaling air- the genitourinary symptoms are: frequent urination, pain when urinating, decreased libido o anorgasmy, premature ejaculation and impotence or erectile dysfunction in men; neurovegetative symptoms are: dry mucus, especially in he mouth and organs which secrete tears, excessive sweating, especially in he face, armpits, hands and soles, sensation of fainting, blush or paleness,- Neurological symptoms are: shaking, itching or paresthesia, stress migraine especially in the nape, contractures, hypersensibility of sounds, intense smell or lights, dizziness o instability; psychophysical symptoms are: fatigue and exhaustion; mental, cognitive or subjective symptoms are: angst, terror, panic, sensation of insecurity, apprehension, preoccupation, feelings of disadvantage or inferiority, inability to face different situations, sensation of being threaten, indecision, difficulty to get concentrated or lack of concentration, apathy, loss of objectivity and capacity to think clearly; the behavioral symptoms are: restlessness and hyperactivity, motor paralysis or immobility, clumsy, shaking, or disrupted movements, tics, stuttering, repetitive pointless movements, avoiding behavior o estrangement of the feared situation.

The emotional response caused by anxiety can occur with the perception or anticipation of a threatening or dangerous situation. Such genetically programmed response accompanies the individuals throughout their lives and its ultimate purpose is o provide protection against potential dangers. A series of body changes occur in the individual during the anxious response, since, when s/he is hypervigilant, heartbeats increase, blood pressure increases, muscular tone increases and respiratory frequency increases. In addition, digestive and sexual function decrease. This set of physical responses prepares the individual to flee, attack or face the threatening situation and are sympathetic-mediated.

*Post-traumatic Stress Disorder* is an anxiety disorder, which can appear after an individual is exposed to a traumatic event, which caused extreme fear, impotence or terror. Post-traumatic Stress Disorder can be produced by personal traumas (for example: rape, war, natural disasters, abuse, serious accidents or captivity) or witnessing or learning about a violent or tragic event.

While it is normal to experience a brief sate of anxiety or depression after said events, people suffering from post-traumatic stress disorder keep "reviving" the trauma; avoiding other people, thoughts or situations relates to the event and present symptoms of excessive emotions.

The symptoms presented by the individuals who suffer from Post-traumatic Stress Disorder can differ greatly, generally, these are included in the following category: 1.- Revival of the experience, wherein the individual frequently have repetitive memories or nightmares on the event, which caused the patient so much angst. Some individuals can have "flashbacks", hallucinations or other emotions and other suffer from strong psychological or physiological stress when certain objects or situations remind them the traumatic event; 2.- *Evasion*: a number of individuals suffering from post-traumatic stress disorder avoid systematically those things that remind them of the traumatic event. This can cause all kinds of evasion: thoughts, feelings or conversations about the incident, as well as activities, places or persons that remind them of the incident. Other individuals don't seem to respond to the things or situations related to the event and they don't remember much about the trauma, showing lack of interest for the activities they enjoyed before the event, isolating from others, experiencing a limited a range of emotions and lacking of expectations about the future; and 3.- *Increased Emotional excitement,* wherein the symptoms present in the individuals suffering from increased emotional excitement can include: Difficulty to get asleep or being incapable of waking up, irritability or sudden of rage, difficulty to get concentrated, state of excessive alert or caution without a specific reason, nervousness o tendency o get frightened.

People suffering from this condition present these symptoms during more than a month and are unable to carry out their functions as they did before the traumatic event. Usually, the symptoms of post-traumatic stress disorder appear during a three-month period after the traumatic situation; however, these symptoms can persist during months or even years after the event.

It is worthy to mention that individuals suffering from post-traumatic stress disorder usually consume alcohol or other drugs as auto-medication in order o feel better. People who suffer from this condition present a high risk of committing suicide.

Nowadays, for the treatment and control of said disorders, anti-depressant such as selective serotonin reuptake inhibitors (SSRI) have been employed, at it has been demonstrated that, in acute episodes characterized by the presence of said pathologies, these drugs reach effectiveness levels of 80% with minimal side effects and are well tolerated by patients. In addition, these can be administered in combination with psychotherapy sessions (especially applying cognitive and behavioral techniques) by psychiatrist.

The concomitant use of anti-depressants combined with anxiolytics, such as benzodiazepine, provides a quicker effect to control the symptoms produced by the aforementioned pathologies; however, one of the main problems which psychiatrist may face is the dependency produced by the use of benzodiazepine during long periods; and on the other hand, there is a risk of interaction between both drug groups, which can cause hepatic damage.

### SUMMARY OF THE INVENTION

In order to provide a pharmaceutical alternative for improving the life quality of patients suffering from diseases such as: major depressive disorder, Obsessive-Compulsive disorder (OCD), panic attacks with or without agoraphobia, generalized anxiety disorders and social phobia, post-traumatic stress disorder, the development of the pharmaceutical composition described below was carried out.

### DETAILED DESCRIPTION OF THE INVENTION

Nowadays, most of the medicines commercially available for the treatment of major depressive disorders and other related diseases comprise active principles which are independently formulated, which have a specific therapeutic activity; however, these medicines can cause severe side effects, as well as a high risk of dependence to the treatment; furthermore, when concomitantly administered, these cause hepatic alterations due o the interaction between them. On the other hand, studies have shown that major depression and anxiety are present in 60% of cases, therefore, it is necessary for psychiatrist to administer a treatment comprising the use of different drugs.

Therefore, in order to reduce all the drawbacks involved when the active principles are independently administered, the development of the pharmaceutical composition which is object of the present invention was carried out, which comprises the synergistic combination of an agent belonging to the triazolobenzodiazepine group and a selective serotonin reuptake inhibitor agent, besides of pharmaceutically acceptable excipients, which produce a satisfactory therapeutic effect when simultaneously administered in a single dosing unit by oral means as opposed to when these are independently administered, providing benefits such as: Less concentrations of the active principles formulated, reduced administered doses, quicker action, increased therapeutic effect efficacy, reduced treatment administration periods and reduced risk of side effects, such as: Dependence on the drugs comprised in the formulation and interaction between the active principles, which could cause hepatic damage.

**Benzodiazepines (BZD)** are a type of drugs with anxiolytic, hypnotic, anticonvulsant, sedative, amnesic and mio-relaxant effects (muscular relaxants).

BZD are nervous-system-depressant agents, which are more selective that other drugs, such as barbituric drugs. Particularly, these drugs act on the limbic, thalamic and hypothalamic system of the central nervous system, down regulating its activity. BZD share a similar chemical structure and have a great affinity with the benzodiazepinic receptor complex found in the central nervous system. Structurally, BZD have a benzene ring with six elements, bound to other diazepine ring with seven elements. Each specific BZD will be generated by the substitution of radicals at different positions.

The specific receptors found in CNS through which BZD exert their effects are part of the benzodiazepine-GABA receptor complex. γ-aminobutiric acid (GABA) is a neurotransmitter having an inhibitory action and its receptors are part of an inhibitory bidirectional system connected between several CNS areas. BZD maximizes the GABA-mediated inhibitory action. GABA acts on several subtypes of receptors called: GABA-A and GABA-B. GABA-A is the subtype of primary receptor found in the CNS through which anxiolytic and sedative drugs act. Certain subtypes of benzodiazepinic receptors seem to be coupled to GABA-A receptors. Three subtypes of benzodiazepinic receptors are known: Those at the cerebellum and brain cortex called BNZ1, those at the brain cortex and the spinal cord called BNZ2 and those at peripheral tissues (such as adrenal glands, kidneys, pineal gland and platelets) called BNZ3. The activation of BNZ1 is a sleep-inducing process, while the drugs binding to BNZ2 cause muscular relaxation, anti-convulsant activity and affect memory. Benzodiazepines bind in a non-specific way to the receptors BNZ1 and BNZ2, maximizing the effects of GABA.

As opposed to other barbiturics, which increase GABA responses, keeping the chlorine channel (C1-) open Turing a Langer period of time, benzodiazepines increase GABA effects, keeping the chlorine channel open in hyperpolarized cells and avoiding a subsequent excitation thereof.

BZD can be categorized according to their action time, being: short action BZD (from 2 to 10 hours) and long action BZD (from 12 to 100 hours) relative to their effect.

In order to administrate BZD, it is important to know if the patient suffers from or has symptoms related to: hepatic diseases, alcoholism, brain diseases, lack of salivation (in children), glaucoma, hyperactivity, kidney or pulmonary diseases, myasthenia gravis, porphyria, pregnancy or sleep apnoea.

During the treatment, convulsions, fever, shaking, muscular weakness, loss of reflexes, intense asthenia, involuntary movements, labored breath, dryness (oral, conjuntival, nasal) of mucus, eritematous skin, hypotension, low blood pressure, mild mental alterations or even confusion and coma. Benzodiazepines cause dependency, which is the reason why these must be used in short treatments. When discontinuing a benzodiazepine treatment, the body breaks free form the addiction in approximately three weeks.

**Alprazolam** is a triazolobenzodiazepine acting as anxiolytic and it is intended for the treatment and control of anxiety disorders, including panic disorder with or without agoraphobia and generalized anxiety, as well as the short-term remission of the typical symptoms of said disorders.

Until now, the accurate action mechanism of Alprazolam has not been established; however, it is know that the agents belonging to the group of 1,4-benzodiazepines exert their action on the CNS by binding to the stereospecific receptors found at several sites of said system. Clinically, all of the benzodiazepines depress the central nervous system relative to the depending dose, from minimal alterations and decreased physical performance to hypnosis.

Alprazolam is quickly absorbed alter being administered by oral means. Maximum plasma concentrations are obtained from 1 to 2 hours after the administration of the corresponding dose. The half-life of Alprazolam plasma elimination is from about 11.2 hours in healthy adult subjects (with a range of 6.3 to 26.9 hr) and of 16.2 hr in healthy elderly subjects. Alprazolam is bound by 80% to human serum proteins (mainly to albumins). Alprazolam metabolism is carried out by hepatic biotransformation through oxidative reactions and glucuronization, which are carried out in the liver by means of microsome oxidation, a process mediated by the cytochrome P450 3A enzymatic system. Its two main metabolites include: alpha-hydroxy-alprazolam, having a biological activity corresponding approximately to half of the therapeutic activity of Alprazolam and a benzofenone, which is essentially inactive. The accumulated plasma levels of these metabolites are extremely low. Alprazolam and its metabolites are mainly eliminated by urine.

Alcoholism, hepatic failure and kidney failure modify Alprazolam pharmacokinetics. In addition, there are pharmacokinetic changes in senile, obese, and alcoholic patients suffering from hepatic disease, healthy women receiving oral contraceptives and cimetidine-treated patients, for which a prolongation of Alprazolam average half-life was reported. Alprazolam cross the placenta barrier and it is excreted through maternal milk. As in the case of other triazolobenzodiazepines, Alprazolam significantly interacts with other drugs, which are metabolized by the hepatic cytochrome P-450.

**Selective serotonin reuptake inhibitors (SRSI)** are the fist class of psychotropic drugs gradually displacing tricyclic anti-depressants. Nowadays, SRSI are considered as first choice drugs, especially in ambulatory patients suffering from mild to moderate depression, as these drugs are as effective as tricyclic anti-depressants; however, these drugs have more favorable adverse effect profile and increased security in term of intoxication cases. Drugs belonging to this group are structurally diverse, which determines rather varied pharmacokinetics.

SRSI are liposoluble drugs having a good absorption alter being administered by oral means. Their absorption rate is slow, more prolonged than that of other types of anti-depressants, usually reaching maximum plasma concentrations about four and eight hours after being administered.

SRSI have low water solubility, which is the reason why these need to bind to plasma proteins in order to be distributed. The high solubility of SRSI determines the quick and wide distribution in the organism, spreading throughout the blood brain barrier.

The degree of fixation of plasma proteins is high (> 90%) for all of the SRSI, being especially high for three of them: Fluoxetine, Paroxetine and Sertraline.

Such drugs are excreted by maternal milk reaching similar concentrations or lesser concentrations than those found in plasma. All of the SRSI are eliminated by the organisms mainly by hepatic metabolism through the microsome system, being excreted by renal means a maximum of 10% of the absorbed dosis of the unchanged drug. This biotransformation process is the main cause for the kinetic differences between the various SRSI.

The main pharmacokinetic interaction is derived from its inhibitor effect on the biotransformation process. SRSI act on the cytochrome P450, which is responsible for the metabolism of a great variety of drugs and, therefore, these can modify the elimination of such drugs when simultaneously administered.

Pharmacokinetic behavior of SRSI is a fundamental element, for the selection and clinical usage of this group of anti-depressants. Undoubtedly, the biotransformation process is of utter importance; t 1/2 values, the formation of active metabolites, the existence of non-linear kinetics and the interaction potential determine the differences between these drugs.

**Paroxetine** is an anti-depressant belonging to the group of selective serotonin reuptake inhibitors (SRSI). This drug is intended for the treatment of depression and major depressive episodes, resides, it has proven to be very useful for the treatment and control of the symptoms related to the Obsessive-Compulsive Disorder (OCD), the panic disorder with or without agoraphobia, the anxiety disorder and social phobia.

It has been proposed that a deficiency in brain 5-hydroxytriptamine (5-HT or Serotonin) is one of the fundamental neurochemical abnormality causing depression. Serotonin is a substance transmitting nervous impulses to the brain and is reduced in depressive states and other nervous disorders.

Paroxetine inhibits serotonin reuptake (5-HT) in a selective way in neurons by competitive interaction in the active transport of neuronal membrane, increasing serotonin concentration in the synaptic space, thus allowing the serotoninergic transmission.

The main metabolites of Paroxetine are products obtained by oxidation and methylation, which conjugated and polar elements quickly eliminated, and due to the absence of pharmacological activity, they are not likely to contribute to the therapeutic action of Paroxetine.

Paroxetine is not structurally or chemically related to tricyclic or tetracyclic or other types of anti-depressants, thus, it has been demonstrated that one of the most important advantages obtained when administering Paroxetine lies in there is a reduced incidence anticholinergic effects and the absence of severe cardio toxicity, and these adverse effects are typical of the group of tricyclic anti-depressants.

Pharmacological and biochemical Studies using animal models in vitro and in vivo have demonstrated the selectivity of 5-HT reuptake in comparison to noradrenergic absorption processes. To that respect, Paroxetine is characterized by a high potency, quick action start, long and continuous duration alter being administered by oral means, as well as increased selectivity when compared to other drugs such as fluoxetine, fluvoxamine and chlorimipramine. Paroxetine does not have pharmacologically active metabolites, as opposed to fluoxetine and sertraline.

Paroxetine is well absorbed by the digestive tract, but it suffers a first-step metabolism. Its oral bioavailability is of 50%. Stable plasma levels are obtained from 7 to 14 days after starting the treatment and pharmacokinetics is not modified during long treatments. It binds by 95% to plasma proteins. More than 95% of the dose is metabolized, mainly in the liver, producing inactive metabolites. Its elimination half-life is of 21 hr (to 36 hr in elderly and severe hepatic failure patients).

It is well known that the cytochrome P450 (CYP) enzimática system is involved in the metabolism of a great Lumber of medicines belonging to different therapeutic classes, including most of anti-depressant agents. The cytochrome P450 enzymatic system is comprised by a group of isoenzymes found in hepatocyte endoplasmic reticulum. Some selective serotonin reuptake inhibitors, which inhibit the CYP, and potentially decrease medicine hepatic clearance are also metabolized by the CYP. The main purpose of the cytochrome P450 system is to catalyze the oxidation of a great variety of chemical substances and transform them into more soluble drugs, which can be easily eliminated. The CYP3A4 is one of the most important isoforms occupying 30% of the total CYP enzymes distributed throughout the body, and it is responsible for the metabolism of about 50% of medicines presenting oxidative biotransformation, including some anti-depressants (Paroxetine) and benzodiazepines (Alprazolam). Most of the CYP is found in the liver; however, there are significant amounts thereof in the small intestine, which play san important role in the first-step metabolism and, as a result, in oral bioavailability of said medicines. Thus, any change in the CYP3A4 activity has clinical implications, especially for those medicines with narrow therapeutic window.

Certain data from in vitro Studies show that the different CYP isoforms are actively involved in Paroxetine and Alprazolam metabolism (CYP2D6 and CYP3A4, respectively) and, therefore, the medicine-medicine non-pure metabolic interaction must be anticipated.

The selective serotonin reuptake inhibitor agent used in the pharmaceutical composition, which is object of the present invention, is the active principle: Paroxetine hemihydrate of Paroxetine hydrochloride, which is present in the formulation in a concentration range of 20.0 mg to 40.0 mg, wherein a concentration of approximately 20.0 mg to 30.0 mg per dosage unit is preferably used.

The triazolobenzodiazepinic agent used in the pharmaceutical composition, which is object of the present invention, is the active principle: Alprazolam, which is present in the formulation in a concentration range of 0.25 mg to 4.0 mg, wherein a concentration of approximately 0.25 mg to 0.50 mg per dosage unit is preferably used.

The pharmaceutical composition protected by the present invention is formulated to be administered by oral means in a single dosing unit in the form of tablets or capsules, which contain the synergistic combination of the active principles: Paroxetine hemihydrate of Paroxetine hydrochloride and Alprazolam, as well as pharmaceutically acceptable excipients.

Said pharmaceutical composition has been developed in order to provide a pharmaceutical alternative for the treatment and control of diseases such as: major depressive episodes, Obsessive-Compulsive disorder (OCD) symptoms, panic attacks with or without agoraphobia, generalized anxiety disorders, social phobia and post-traumatic stress disorder, which provides important benefits such as: reduced concentrations of at least one of the active principles contained in the formulation, reduced administered doses, quicker action, increased therapeutic effect efficacy, reduced treatment administration periods, satisfactory control of the symptoms suffered by patients presenting said diseases and decreased possibility of presenting adverse effects, including: Dependence on the drugs comprised in the formula and interaction between both active principles, which could cause hepatic damage.

In order to assess the efficiency and tolerance of the pharmaceutical composition object of the present invention, as well as the synergetic effect and the positive interaction between the active principles, Paroxetine hemihydrate and Alprazolam, combined in a single dosage unit, a comparative clinical study was conducted, wherein the above active principles were individually administered, as well as the combination thereof.

### INTERACTION STUDY BETWEEN PAROXETINE HEMIHYDRATE AND ALPRAZOLAM IN HEALTHY VOLUNTEERS

25 voluntary healthy subjects were included in the placebo-controlled double blind study with repeated doses (15 days) in 4 periods of crossover study.

The volunteers were subjected to different trials before starting the study, including clinical interview, physical exam and electrocardiographic, hematological, and chemical tests, urinalysis. Volunteer women had an effective contraceptive method and were subjected to a beta-sub-unity pregnancy test, which had to be negative.

Each subject received 4 random treatment sequences: 1.- Paroxetine hemihydrate/Placebo. 2.- Alprazolam/Placebo. 3.- Paroxetine hemihydrate/Alprazolam. 4.- Placebo/Placebo.

The study subjects were randomly allocated to one of the 4 possible treatment sequences. In each treatment sequence, the study medicine was administered by the morning, once a day during 15 consecutive days. The subjects fasted until 2 hours after administrating the dose. There was a week of treatment-free window between the treatment sequences. On Day 13 and 15 of each treatment sequence, pre-dose blood samples were taken in order to ensure the plasmatic concentrations of Paroxetine hemihydrate and Alprazolam reached a stable level. Serial blood samples were taken day 15, 0.5, 1, 1.5, 2, 2.5, 3, 3.5, 4, 5, 6, 8, 10 and 12 hours after the dose in order to measure the plasmatic concentrations of Paroxetine hemihydrate and Alprazolam. A single daily sample was taken in the morning of day 16 and 19.

On day 1 and 15 of each treatment sequence, the influence of the different treatment sequences over the pharmacodynamic effects and mood status assessed before the dose 1, 2, 4 and 8 hours alter the administration of the dose, using a testing set of Psychomotor Function and Visual Analog Scale (VAS), the latter is focused on mood variables.

A daily record of adverse events and vital signs was evaluated, as well as the laboratory security parameters; the clinical efficacy was also evaluated during the pre-study visit and during the last study visit.

## Claims

1. A pharmaceutical composition comprising the synergistic combination of Paroxetine hemihydrate of Paroxetine Hydrochloride and Alprazolam, as well as pharmaceutically acceptable excipients, in which the amount of said Paroxetine hemihydrate of Paroxetine Hydrochloride is of 20.0 mg to 40.0 mg and the amount of said Alprazolam is of 0.25 mg to 4.0 mg, said composition being formulated in a single dosage unit to be administered orally for use in the treatment and control of major depressive episodes, symptoms of Obsessive-Compulsive Disorder, panic attacks, generalized anxiety disorders, social phobia and post-traumatic stress disorder.

2. The pharmaceutical composition for use according to claim 1, **characterized in that** said amount of Paroxetine hemihydrate of Paroxetine Hydrochloride is of 20.0 mg to 30.0 mg per dosage unit.

3. The pharmaceutical composition for use according to claim 1, **characterized in that** said amount of Alprazolam is of 0.25 mg to 0.50 mg per dosage unit.

4. The pharmaceutical compositionfor use according to anyone of claims 1 to 3, **characterized in that** the amount of Paroxetine hemihydrate of Paroxetine Hydrochloride is of 20.0 mg and the amount of Alprazolam is of 0.25 mg per dosage unit.

5. The pharmaceutical composition for use according to anyone of claims 1 to 4, **characterized in that** said single dosage unit is in the form of a capsule or a tablet.

6. The pharmaceutical composition for use according to anyone of claims 1 to 5, **characterized in that** said panic attacks are panic attacks with agoraphobia.

## Patentansprüche

1. Pharmazeutische Zusammensetzung umfassend die synergistische Kombination von Paroxetin-Hydrochlorid-Hemihydrat und Alprazolam, sowie pharmazeutisch verträgliche Hilfsstoffe, in welcher die Menge an Paroxetin-Hydrochlorid-Hemihydrat 20,0 mg bis 40,0 mg und die Menge an Alprazolam 0,25 mg bis 4,0 mg beträgt, wobei die Zusammensetzung in einer Einzeldosierungseinheit zur oralen Verabreichung zur Verwendung bei der Behandlung und Kontrolle von längeren depressiven Phasen, Symptomen von Zwangsstörungen, Panikattacken, allgemeinen Angststörungen, sozialen Phobien und posttraumatischen Belastungsstörungen formuliert ist.

2. Pharmazeutische Zusammensetzung zur Verwendung nach Anspruch 1, **dadurch gekennzeichnet, dass** die Menge an Paroxetin-Hydrochlorid-Hemihydrat 20,0 mg bis 30,0 mg pro Dosierungseinheit beträgt.

3. Pharmazeutische Zusammensetzung zur Verwendung nach Anspruch 1, **dadurch gekennzeichnet, dass** die Menge an Alprazolam 0,25 mg bis 0,50 mg pro Dosierungseinheit beträgt.

4. Pharmazeutische Zusammensetzung zur Verwendung nach einem der Ansprüche 1 bis 3, **dadurch gekennzeichnet, dass** die Menge an Paroxetin-Hydrochlorid-Hemihydrat 20,0 mg und die Menge an Alprazolam 0,25 mg pro Dosierungseinheit beträgt.

5. Pharmazeutische Zusammensetzung zur Verwendung nach einem der Ansprüche 1 bis 4, **dadurch gekennzeichnet, dass** die Einzeldosierungseinheit in Kapsel- oder Tablettenform vorliegt.

6. Pharmazeutische Zusammensetzung zur Verwendung nach einem der Ansprüche 1 bis 5, **dadurch gekennzeichnet, dass** es sich bei den Panikattacken um Panikattacken mit Agoraphobie handelt.

## Revendications

1. Une composition pharmaceutique comprenant la combinaison synergique d'hémihydrate de paroxétine, de chlorhydrate de paroxétine et d'alprazolam, ainsi que des excipients pharmaceutiquement acceptables, dans laquelle la quantité de ladite hémihydrate de paroxétine 5 de chlorhydrate paroxétine est de 20,0 mg à 40,0 mg et la quantité du dit alprazolam est de 0,25 mg à 4,0 mg, ladite composition étant formulée en une seule unité de dosage à administrer par voie orale dans le traitement et le contrôle d'épisodes dépressifs majeurs, des symptômes de trouble obsessionnel-compulsif, de crises de panique, des troubles d'anxiété généralisée, de phobie sociale et de stress post-traumatique.

2. Composition pharmaceutique pour une utilisation selon la revendication 1, **caractérisée en ce que** ladite quantité d'hémihydrate de paroxétine de chlorhydrate de paroxétine est de 20,0 mg à 30,0 mg par dose unitaire.

3. Composition pharmaceutique pour une utilisation selon la revendication 1, **caractérisée en ce que** ladite quantité d'alprazolam est de 0,25 mg à 0,50 mg par dose unitaire.

4. Composition pharmaceutique pour une utilisation selon l'une des revendications 1 à 3, **caractérisée en ce que** la quantité d'hémihydrate de paroxétine de chlorhydrate de paroxétine est de 20,0 mg et la quantité d'alprazolam est de 0,25 mg par dose unitaire.

5. Composition pharmaceutique pour une utilisation selon l'une des revendications 1 à 4, **caractérisée en ce que** ladite dose unitaire se présente sous forme d'une gélule ou d'un comprimé 20.

6. Composition pharmaceutique pour une utilisation selon l'une des revendications 1 à 5, **caractérisée en ce que** lesdites crises de panique sont des crises de panique avec agoraphobie.
